(19) Europäisches Patentamt
European Patent Office
Office européen des brevets

(11) **EP 3 554 378 B1**

(12) **EUROPEAN PATENT SPECIFICATION**

(45) Date of publication and mention
of the grant of the patent:
**28.07.2021 Bulletin 2021/30**

(51) Int Cl.:
*A61B 8/06* (2006.01)     *A61B 8/08* (2006.01)
*A61B 8/00* (2006.01)

(21) Application number: **17821582.8**

(22) Date of filing: **18.12.2017**

(86) International application number:
**PCT/EP2017/083397**

(87) International publication number:
**WO 2018/114858 (28.06.2018 Gazette 2018/26)**

(54) **ULTRASONIC TRANSDUCER ARRAY MONITORING DURING TRANSCRANIAL ULTRASOUND PROCEDURES**

ULTRASCHALLWANDLER-ARRAY-ÜBERWACHUNG BEI TRANSKRANIELLEN ULTRASCHALLVERFAHREN

SURVEILLANCE DE RÉSEAU DE TRANSDUCTEURS ULTRASONORES PENDANT DES PROCÉDURES ULTRASONORES TRANSCRÂNIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2016 US 201662436164 P**

(43) Date of publication of application:
**23.10.2019 Bulletin 2019/43**

(73) Proprietor: **Koninklijke Philips N.V.**
**5656 AG Eindhoven (NL)**

(72) Inventors:
• **SUTTON, Jonathon, Thomas**
 **5656 AE Eindhoven (NL)**
• **SHI, William, Tao**
 **5656 AE Eindhoven (NL)**
• **POWERS, Jeffry, Earl**
 **5656 AE Eindhoven (NL)**
• **SEIP, Ralf**
 **5656 AE Eindhoven (NL)**

(74) Representative: **Philips Intellectual Property & Standards**
 **High Tech Campus 5**
 **5656 AE Eindhoven (NL)**

(56) References cited:
**WO-A1-2006/068103     WO-A2-02/45572**
**WO-A2-2014/207665     US-A1- 2002 103 436**
**US-B2- 8 211 023**

**Description**

Technical Field

This invention is set out in the appended claims and generally relates to medical ultrasound systems and, in particular, to ultrasound systems which perform imaging and therapy procedures.

Background

Ischemic stroke is one of the most debilitating disorders known to medicine. The blockage of the flow of blood to the brain can rapidly result in paralysis or death. Attempts to achieve recanalization through thrombolytic drug therapy such as treatment with tissue plasminogen activator (tPA) has been reported to cause symptomatic intracerebral hemorrhage in a number of cases. Advances in the diagnosis and treatment of this crippling affliction are the subject of continuing medical research.

US Pat. 8,211,023 (Swan et al.) describes an ultrasound system which provides microbubble-mediated therapy to a thrombus such as one causing ischemic stroke. Microbubbles are infused, delivered in a bolus injection, or developed in the bloodstream and flow to the vicinity of a thrombus. Ultrasound energy is delivered to the microbubbles at the site of the thrombus to disrupt or rupture the microbubbles. This energetic microbubble activity can in many instances aid in dissolving or breaking up the blood clot and return a nourishing flow of blood to the brain and other organs. Such microbubble activity can be used to deliver drugs encapsulated in microbubble shells, as well as microbubble-mediated sonothrombolysis. The Swan et al. patent shows the ultrasonic energy being delivered for sonothrombolysis from an ultrasound array probe controlled by an ultrasound system.

Summary

The present invention is defined by the independent claim. Advantageous embodiments are provided in the dependent claims.

The present invention is driven from a recognition that, in clinically safe and effective sonothrombolysis treatments, the ultrasound array probe(s) delivering the ultrasound energy to the clot target region should be continuously effectively acoustically coupled to the head of the patient and continually aimed at the target volume. The target volume may include, for example, the thrombus and in some instances the surrounding area. However, a continuous coupling cannot be maintained if the transducer delivering the ultrasound has moved or been disturbed from effective acoustic coupling with the head of the patient. During traditional ultrasound imaging examinations, this coupling is monitored by observing the resulting image, which is not always convenient during a therapy procedure and is not available in non-imaging ultrasound procedures. Accordingly, it is desirable to monitor the probe placement on the head of the patient to assure that these conditions are continuously met during treatment.

While monitoring the transducer coupling is desirable, existing techniques are impractical. For example, real-time monitoring of device positioning with 3D CT or fluoroscopy is cumbersome and resource intensive. Magnetic resonance imaging is also cumbersome, offers poor temporal resolution, and restricts *ad hoc* point-of-care procedures and patient monitoring during its long scan times. Ancillary devices to monitor the relative position of the device and the skull, such as accelerometers or electromagnetic/ acoustic positioning devices, can be insensitive to small changes in angle of incidence or coupling material failure.

Accordingly, it is an object of the present invention to monitor the acoustic coupling of a therapy transducer to the head of a patient to assure that it is continuously effectively acoustically coupled to the head of the patient.

It is a further object of the present invention to monitor transducer placement on the head of a patient during therapy to assure that the transducer has not moved during treatment.

The present invention pertains to an ultrasound system adapted to perform therapy on the head of a subject and comprising: an array of transducer elements, configured to acoustically couple to the head of a subject, and adapted to transmit therapeutic ultrasonic energy toward a therapy site in the head of the subject, and further comprising a motion detecting transducer configured to receive echo signals from the head of the subject at a first time and a second time; and a processor configured to analyze, during therapy, the echo signals received at the first and second times to determine whether transducer motion has occurred from the first time to the second time, wherein the processor further comprises a motion detecting circuit configured to analyze the echo signals received at the first and second times by comparison or correlation. technique can be applied to any transcranial ultrasound procedure, including *inter alia* blood-brain barrier disruption, thermal ablation, non-imaging monitoring techniques, and neuromodulation.

In the drawings:

FIGURE 1 illustrates in block diagram form an ultrasonic diagnostic imaging and therapy system for ultrasonic transcranial therapy.

FIGURE 2 illustrate the delivery of sonothrombolysis therapy in a two-dimensional (2D) imaging plane

FIGURE 3 illustrates the delivery of sonothrombolysis therapy in a three-dimensional image volume in conjunction with monitoring of transducer coupling and positioning in accordance with the principles of the present invention.

FIGURE 4 illustrates a probe and headset for sonothrombolysis therapy modeled on the head of a mannequin.

FIGURE 5 shows a typical waveform received by a transducer position monitoring transducer element in accordance with the principles of the present invention.

FIGURE 6a illustrates in block diagram form a first implementation of a transducer coupling and position monitoring subsystem in accordance with the present invention.

FIGURE 6b illustrates in block diagram form a preferred implementation of a transducer coupling and position monitoring subsystem in accordance with the present invention.

FIGURE 7 illustrates a method which does not form part of the present invention, for monitoring transducer positioning during a therapy procedure.

FIGURE 8 illustrates the insonification of the cranium of a subject, the reception of transducer motion monitoring signals, and the correlation of those signals before and after transducer motion has occurred.

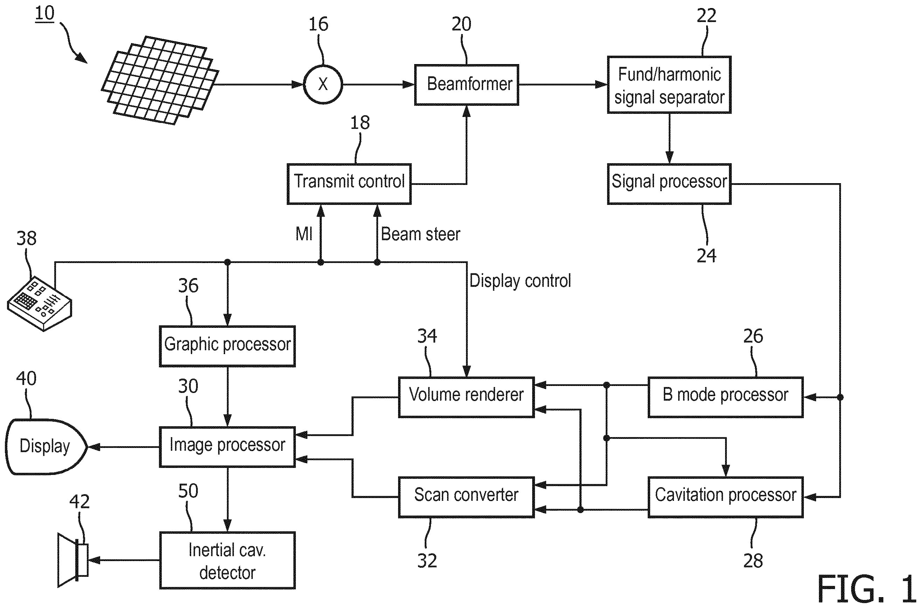

Referring to FIGURE 1, an ultrasound system constructed in accordance with the principles of the present invention is shown in block diagram form. It is understood that systems of the invention may include one or more transducer arrays included in one or more probes, as described hereinafter. A transducer array 10 is provided for transmitting ultrasonic waves for therapy and other uses as described below and receiving echo information. In certain embodiments, the transducer array is a 2D transducer array. In the implementation of FIGURE 1 the array is shown as a two-dimensional array of transducer elements capable of steering therapeutic waves in three dimensions and providing 3D images and other information. The array is located in an ultrasound probe which mounts on a headset that locates the array in acoustic contact with the temple on the side of the head for transcranial delivery of sonothrombolysis. The elements of the array are coupled to a transmit/ receive (T/R) switch 16 which switches between transmission and reception and protects the receive channels of the system beamformer 20 from high energy transmit signals. The transmission of ultrasonic pulses from the transducer array 10 is directed by the transmit controller 18 coupled to the beamformer 20, which receives input from the user's operation of the user interface or control panel 38.

The echo signals received by elements of the array 10 are coupled to the system beamformer 20 where the signals are combined into coherent beamformed signals. For example, the system beamformer 20 in this example has 128 channels, each of which drives an element of the array to transmit energy for therapy or imaging, and receives echo signals from one of the transducer elements. In this way, the array is controlled to transmit steered beams of energy and to steer and focus received beams of echo signals.

The beamformed receive signals are coupled to a fundamental/harmonic signal separator 22. The separator 22 acts to separate linear and nonlinear signals so as to enable the identification of the strongly nonlinear echo signals returned from microbubbles or tissue and, for the present invention, fundamental frequency signals for detection of probe movement. The separator 22 may operate in a variety of ways such as by bandpass filtering the received signals in fundamental frequency and harmonic frequency bands (including super-, sub-, and/or ultra-harmonic signal bands), or by a process for fundamental frequency cancellation such as pulse inversion or amplitude modulated harmonic separation. Other pulse sequences with various amplitudes and pulse lengths may also be used for both linear signal separation and nonlinear signal enhancement. A suitable fundamental/harmonic signal separator is shown and described in international patent publication WO 2005/074805 (Bruce et al.) The separated fundamental and/or nonlinear (harmonic) signals are coupled to a signal processor 24 where they may undergo additional enhancement such as speckle removal, signal compounding, and filtering for noise elimination.

The processed signals are coupled to a B mode processor 26 and a cavitation processor 28. The B mode processor 26 employs amplitude detection for the imaging of structures in the body such as muscle, tissue, and blood cells. B mode images of structure of the body may be formed in either the harmonic mode or the fundamental mode. Tissues and microbubbles in the body both return both types of signals and the stronger harmonic returns of microbubbles enable microbubbles to be clearly segmented in an image in most applications. A cavitation processor 28 detects signal characteristics of cavitation and produces cavitation image and alert signals as described below. For example, subharmonic and ultraharmonic returns only from microbubbles further enable microbubbles to be clearly segmented in an image. The system may also include a Doppler processor which processes temporally distinct signals from tissue and blood flow for the detection of motion of substances in the image field including blood cells and microbubbles. The anatomic and cavitation signals produced by these processors are coupled to a scan converter 32 and a volume renderer 34, which produce image data of tissue structure, blood flow, microbubble cavitation, or a combined image of several of these characteristics. The scan converter converts echo signals with polar coordinates into image signals of the desired image format such as a sector image in Cartesian coordinates. The volume renderer 34 converts a 3D data set into a projected 3D image as viewed from a given reference point as described in US Pat. 6,530,885 (Entrekin et al.)

As described therein, when the reference point of the rendering is changed the 3D image can appear to rotate in what is known as kinetic parallax. This image manipulation is controlled by the user as indicated by the Display Control line between the user interface 38 and the volume renderer 34. Also described is the representation of a 3D volume by planar images of different image planes, a technique known as multiplanar reformatting. The volume renderer 34 can operate on image data in either rectilinear or polar coordinates as described in US Pat. 6,723,050 (Dow et al.) The 2D or 3D images are coupled from the scan converter and volume renderer to an image processor 30 for further enhancement, buffering and temporary storage for display on an image display 40.

A graphics processor 36 is also coupled to the image processor 30 which generates graphic overlays for displaying with the ultrasound images. These graphic overlays can contain standard identifying information such as patient name, date and time of the image, imaging parameters, and the like, and can also produce a graphic overlay of a beam vector steered by the user as described below. For this purpose the graphics processor receives input from the user interface 38. In an example, which does not form part of the present invention, the graphics processor can be used to overlay a cavitation image over a corresponding anatomical B mode image. The user interface is also coupled to the transmit controller 18 to control the generation of ultrasound signals from the transducer array 10 and hence the images produced by and therapy applied by the transducer array. The transmit parameters controlled in response to user adjustment include the MI (Mechanical Index) which controls the peak intensity of the transmitted waves, which is related to cavitational effects of the ultrasound, and steering of the transmitted beams for image positioning and/or positioning (steering) of a therapy beam as discussed below.

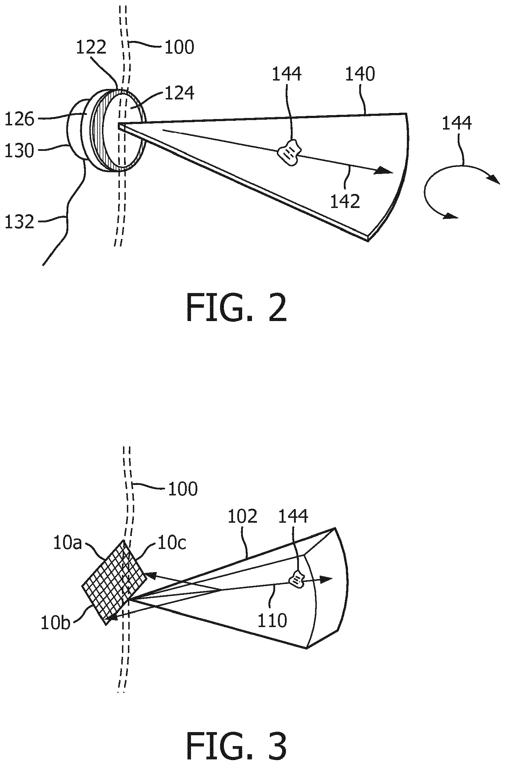

FIGURE 2 illustrates the conduct of sonothrombolysis in two dimensions with a one-dimensional transducer array. In this example the transducer array 122 is a one-dimensional array which performs 2D imaging. This transducer array, like the other arrays described herein, is covered with a lens 124 which electrically insulates the patient from the transducer array and in the case of a one-dimensional array may also provide focusing in the elevation (out-of-plane) dimension. The lens is pressed against the skinline 100 for acoustic coupling to the patient. The transducer array 122 is backed with air or acoustic damping material 126 which attenuates acoustic waves emanating from the back of the array to prevent their reflection back into the transducer elements. Behind this transducer stack is a device 130 for rotating the image plane 140 of the array. The device 130 may be a simple knob or tab which may be grasped by the clinician to manually rotate the circular array transducer in its rotatable transducer mount (not shown). The device 130 may also be a motor which is energized through a conductor 132 to mechanically rotate the transducer as discussed in US Pat. 5,181,514 (Solomon et al.) Rotating the one-dimensional array transducer 122 as indicated by arrow 144 will cause its image plane 140 to pivot around its central axis, enabling the repositioning of the image plane for full examination of the vasculature in front of the transducer array. As discussed in the '514 patent, the planes acquired during at least a 180° rotation of the array will occupy a conical volume in front of the transducer array, which may be rendered into a 3D image of that volumetric region. Other planes outside this volumetric region may be imaged by repositioning, rocking or tilting the transducer array in its headset in relation to the skull beneath the skinline 100. If a stenosis, a blood clot 144, is found in the image of the plane being imaged, the therapeutic beam vector graphic 142 can be steered by the clinician to aim and focus the beam at the stenosis 144 and therapeutic pulses applied to disrupt the microbubbles at the site of the stenosis to lyse the obstruction.

FIGURE 3 illustrates a 3D imaging/therapy implementation of the present invention which uses a 2D matrix array transducer 10a. In this illustration the insulating lens (not shown) covering the transducer array 10a is held against the skinline 100 of the patient with the imaged volume 102 inside the skull being diagnosed and treated. The user will see a 3D image of the volume 102 on the display 40 of the ultrasound system in either a multiplanar or volume rendered 3D projection. The user can manipulate the kinetic parallax control to observe the volume rendered 3D image from different orientations. The user can adjust the relative opacity of the tissue and flow components of the 3D image to better visualize the vascular structure inside the brain tissue as described in US Pat. 5,720,291 (Schwartz) or can turn off the B mode (tissue) portion of the display entirely and just visualize the flow of the vascular structure inside the 3D image volume 102.

When the site of the treatment such as a thrombus 144 is being imaged in the volume 102, a microbubble contrast agent is introduced into the patient's bloodstream. In a short time the microbubbles in the bloodstream will flow to the vasculature of the treatment site and appear in the 3D image. Therapy can then be applied by agitating or breaking microbubbles at the site of the stenosis in an effort to dissolve the blood clot. The clinician activates the "therapy" mode, and a therapy graphic 110 appears in the image field 102 on the display, depicting the vector path of a therapeutic ultrasound beam with a graphic thereon which may be set to the depth of the thrombus. The therapeutic ultrasound beam is manipulated by a control on the user interface 38 until the vector graphic 110 is focused at the site of the blockage. The energy produced for the therapeutic beam can be within the energy limits of diagnostic ultrasound or in excess of the ultrasound levels permitted for diagnostic ultrasound. The energy of the resulting microbubble ruptures will strongly agitate a blood clot, tending to lyse the clot and dissolve it in the bloodstream. In many instances insonification of the microbubbles at diagnostic energy levels will be sufficient to dissolve the clot. Rather than breaking in a single event, the microbubbles may be vibrated and oscillated, and the energy from such extended oscillation prior to dissolution of the microbubbles can be sufficient to lyse the clot. When vigorous activity of the microbubbles is desired to quickly lyse a blood clot or rapidly break up a large clot, it may be decided to induce desirable cavitation at the site of the blockage to stimulate this activity. Inertial cavitation will produce the most vigorous activity, while stable cavitation will produce a lower level of microbubble agitation. The presence of cavitation at the site of the occlusion and its type is detected by a cavitation detector 50, which analyzes characteristics of echo signals to determine whether cavitation is occurring and, if so, the type of cavitation. The two different forms of cavitation produce ultrasonic backscatter of different characteristics. Stable cavitation produces a strong subharmonic and/or ultraharmonic response, while unstable, or inertial cavitation produces broadband noise. The cavitation detector analyzes returning echo signals for indications of these characteristics and informs the clinician when the required type of cavitation is identified, e.g., by coloring the site of the therapy in an ultrasound image with a color where adequate cavitation has been identified. If the signature for inertial cavitation is detected, for example, and stable cavitation is desired, the inertial cavitation detector 50 causes speaker 42 to issue an alarm. The user responds to this information by reducing the ultrasound output power (MI) being generated by the sonothrombolysis array. If cavitation is not detected at all, for example, by no indication of cavitation coloring of the site of the occlusion in the image, then the output power of the sonothrombolysis array is increased until cavitation is detected. This output power scaling can be accomplished automatically without user intervention via an output power control loop, for example. The treatment is continued at the appropriate setting. Such usage allows the system to compensate for the attenuation generated by different temporal bone windows and any varying attenuation due to different acoustic properties of brain tissue.

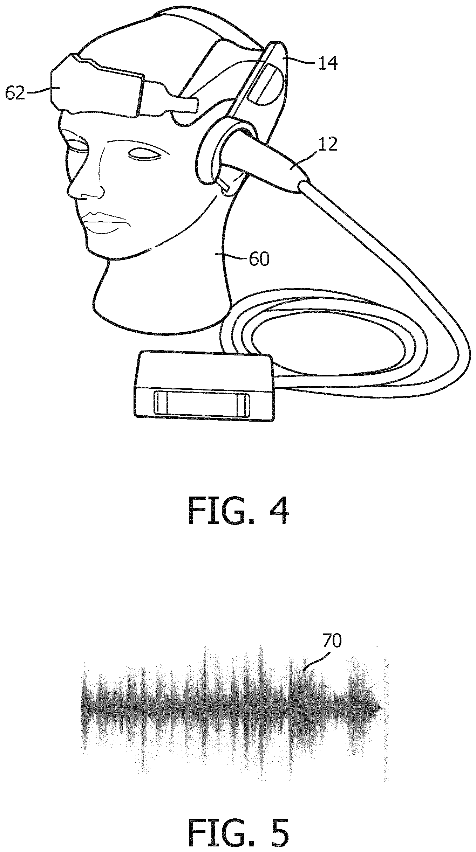

In accordance with the principles of the present invention, one or more elements of the array 10a are used to receive ultrasonic scattering from a transmit beam and process the received signals to detect whether there has been any probe motion during therapy. In FIGURE 3 elements 10b and 10c on opposite sides of the array are shown receiving backscattered ultrasound from a beam transmission along vector 110 as indicated by the arrows directed back to elements 10b and 10c from the vector. A typical backscatter signal 70 produced by one of these transducer elements is shown in FIGURE 5. The backscatter signal may be received over the full depth of field of the image region 102, but preferably reception is gated over a selected depth of field. Desirably, the depth of field should include one or more landmarks that do not move during treatment and can be identified as indicia that the probe has remained stationary relative to the head. In general, landmarks may include any static tissue or bone of the head, as opposed to circulating blood or bubbles. This can be done by filtering or otherwise removing time-varying signals such as those returned by flowing microbubbles, for instance. Undesired strong signals returned from flowing microbubbles can be further eliminated by using only fundamental frequency signals of the backscatter for probe motion detection, which avoids reception of the strong nonlinear (harmonic) signals returned from microbubbles. A preferred gated range is one that detects only near-field scattering returned as the transmit beam passes through the skull, which will be repeatable from one transmit-receive interval to another so long as the transducer has not moved or lost some or all of its acoustic coupling with the head. Echoes returned from the near field thus use the skull bone as the landmark.

FIGURE 4 illustrates a headset 62 for a sonothrombolysis array probe 12 of the present invention mounted on the head 60 of a mannequin. The sides of the head of most patients advantageously provide suitable acoustic windows for transcranial ultrasound at the temporal bones around and in front of the ears on either side of the head. In order to transmit and receive echoes through these acoustic windows the transducer arrays must be in good acoustic contact at these locations which may be done by holding the transducer arrays against the head with the headset 62. An implementation, which does not form part of the present invention, may have a snap-on deformable acoustic standoff which allows the transducer array to be manipulated by its conformal contact surface and aimed at the arteries within the brain while maintaining acoustic contact against the temporal window. An array 10 is integrated into the probe housing 12 which allows it to address the requirements of stable positioning and tight coupling to the patient's temporal bone. The illustrated probe housing is curved by bending the probe handle by 90°, which makes the probe more stable when attached to the headset 62. The acoustic coupling objective is facilitated by integrating a mating spherical surface into the probe handle, which allows it to pivot in the headset 62 until it is strongly and tightly coupled to the temporal window of the patient.

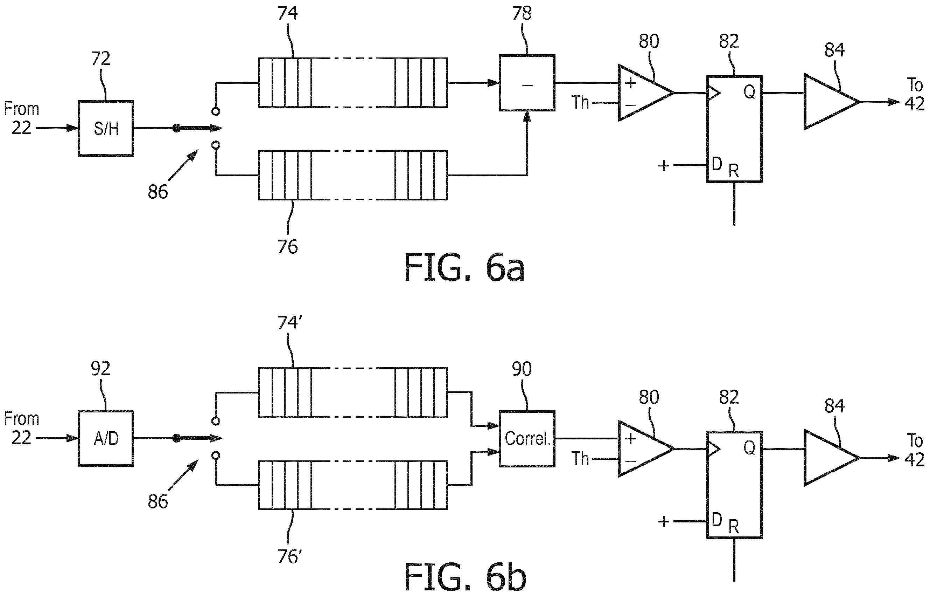

Circuitry which processes signals from a motion detecting transducer element such as elements 10b and 10c in FIGURE 3 is shown in FIGURES 6a and 6b. FIGURE 6a shows an analog implementation in which the fundamental frequency signals from a motion monitoring element such as that shown in FIGURE 5 are coupled from the fundamental/harmonic signal separator 22 to a sample-and-hold circuit 72. The signal which is sampled by this circuit may be the detected envelope of the r.f. waveform, as it is not usually necessary to use the r.f. signal. Samples of the signal 70 are coupled to an analog signal storage device 74 or 76, such as an analog shift register or an analog random access memory (ARAM), in accordance with the setting of a signal steering device shown as single pole, double throw switch 86. At the beginning of therapy, when the transducer array 10 is properly coupled to the head of the patient and aimed at the therapy site, samples of the signal received by the monitoring transducer element 10b or 10c are steered to the storage device 76 and stored to be used as a reference. Since the monitoring transducer elements 10b and 10c are elements of the same array 10a used for therapy, they are positionally related to the therapy elements in a fixed relationship. Thus, any motion experienced by elements 10b or 10c in relation to the patient will also be experienced by the therapy array elements. Thereafter, during treatment, signals are received by the monitoring transducer element(s) from time to time and samples are steered by switch 86 to storage device 74. Preferably, the current signal sample is correlated with the reference signal, or the previous signal, and compared to a threshold value to decide whether there has been transducer motion. Algorithmically, this correlation and decision-making process can be expressed as:

$$\text{RF line } 0 + \text{RF line } 1 = \text{correlation coefficient} + \text{phase} \rightarrow \text{decision } 1$$

$$\text{RF line } 1 + \text{RF line } 2 = \text{correlation coefficient} + \text{phase} \rightarrow \text{decision } 2$$

In an alternative embodiment, the reference and current signal samples stored in storage devices 74 and 76 are clocked or read out of the storage devices in depth-corresponding unison and the reference samples previously stored in storage device 76 are subtracted from the samples most recently stored in storage device 74 by a subtraction circuit 78 and the magnitude of the difference is applied to one input of a comparator 80, where the difference is compared to a threshold Th. So long as the magnitude of the difference is less than the threshold value, no output value is produced by the comparator 80. This will be the case when there has been no movement of the transducer 10 or acoustic decoupling from the head of the patient. When there has been no transducer movement or acoustic decoupling, the echo signals received at the two time intervals from identical transmit pulses will have traveled through the same tissue paths and be the same. Thus, the subtraction of the two (after allowing for signal noise) will be at or near a zero magnitude, indicating no movement or decoupling. But when there has been movement or decoupling, the later-received signals will differ from the reference signals and their difference will have a magnitude which will exceed the threshold Th. This will cause the comparator 80 to produce an output signal that will clock D-type flip-flop 82. In some procedures, particularly those performed with a non-imaging system, the setting of the flip-flop 82 can also cause a pause in the application of ultrasonic therapy. The now-set Q output of the flip-flop causes drive circuit 84 to produce an audio signal, which drives loudspeaker 42 to issue an alarm alerting a clinician. The clinician will then examine the headset and the ultrasound image to see if the headset needs to be adjusted on the head of the patient or the transducer treatment vector 110 re-aimed at the thrombus being lysed.

The motion detection circuit of FIGURE 6b is similar to that of FIGURE 6a except that, instead of taking the difference between the current and reference waveforms 70, the two waveforms are cross-correlated by a correlator 90. The correlator 90 produces a cross-correlation estimate which is a quantitative estimate of the similarity of the instant and reference signals in question. Strong decreases in the cross-correlation estimate, indicative of transducer motion or acoustic decoupling, will exceed the threshold value Th, causing the D flip-flop 82 to be set and the drive circuit 84 to cause the loudspeaker 42 to issue an audible alert. The implementation of FIGURE 6b also illustrates a digital implementation of a motion detection circuit. The signals 70 received from the signal separator 22 are digitally sampled by an A/D converter 92 and directed by steering circuit 86 to one of two digital storage devices 74' and 76', which may be digital shift registers or digital memories, for instance. Correlation is then estimated in the digital domain.

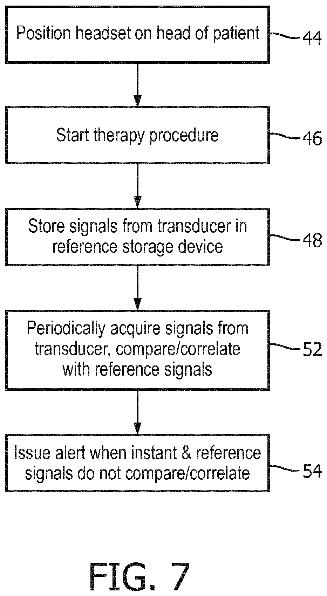

FIGURE 7 illustrates a method, which does not form part of the present invention, for using the ultrasound system of FIGURES 1, 4, 6a, 6b for a clinical therapy procedure. In step 44 the headset 62 containing the therapy and motion detecting transducer elements is properly positioned on the head of the patient, with the transducer acoustically coupled to the head and aimed at the therapy region in the skull. Thereafter the therapy procedure is started in step 46. In step 48 the signal received by one or more motion detecting transducer elements is stored in the storage device(s) for reference signals. As the therapy procedure continues, signals received by the motion detecting transducer element(s) are acquired from time to time and compared or correlated with the reference signals acquired at the outset of the procedure. If the comparison/ correlation shows no significant variance in the signals previously and currently acquired, the procedure continues. But if the two temporally different signals do not compare or correlate, an alert is issued by the system in step 54 to summon a clinician to check on the positioning of the transducer.

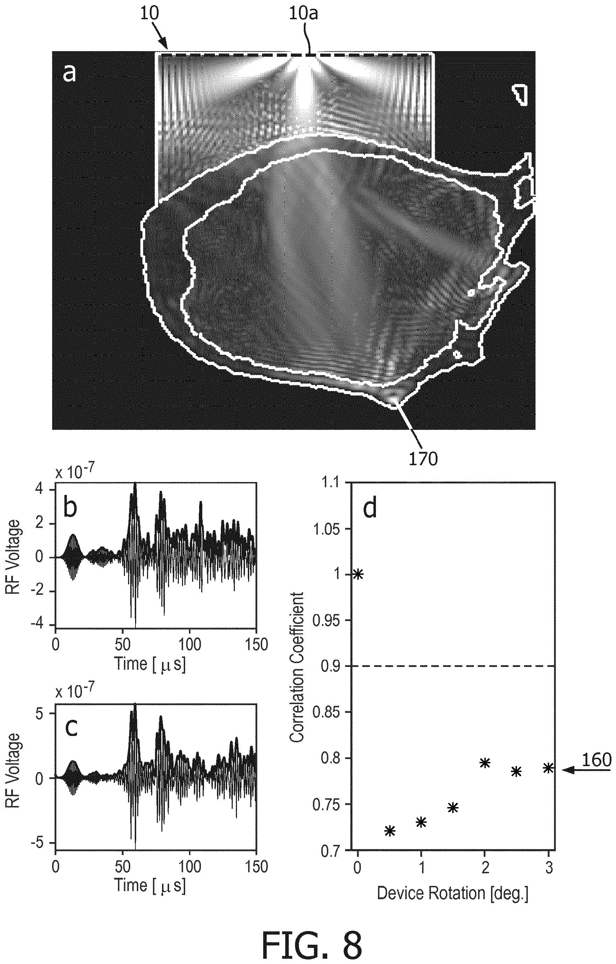

FIGURE 8 illustrates signals and correlation results from a reduction to practice of the present invention. Box a illustrates the cranium 170 of a subject, a dog in this instance, which is insonified by ultrasound from an element of transducer array 10. Ultrasound is transmitted and received by a motion monitoring transducer element 10a of the array as shown by the bright areas of ultrasonic energy in box a. A motion monitoring transducer element can be one of the transducer elements of the therapy array, or it can be a transducer element separate from the therapy array but positionally related thereto. The echo signal received by element 10a during one motion monitoring interval is shown in box b. If this transmission and reception is repeated at a subsequent time with no intervening transducer motion, a virtually identical echo signal will be received as shown in box b, and the correlation of the earlier and later received signals will produce a correlation coefficient of "1", as shown in the upper half of box d. In this experiment, a signal was again transmitted and received by element 10a after rotation of the array 10 in increments totaling 3°, which is shown in box c. The correlation of the earlier (box b) and later (box c) signals resulted in correlation coefficients ranging from 0.7 to 0.8 as indicated at 160. Since these correlation coefficients are below a pre-determined threshold of 0.9 (indicated by the dashed line in box d), they are indicative of unacceptable transducer motion and an alert is produced by the ultrasound system.

It is thus seen from the foregoing that a system of the present invention comprises a motion detecting transducer acoustically coupled to the head of a subject and a processing unit coupled to a storage device which stores instructions which, when executed by the processing unit, cause the processing unit to a) receive echo signals reflected from the head of the subject at a first time and at a second time; b) filter (*e.g.*, by signal processor 24) time-varying signals from the echo signals, thereby providing a first signal signature corresponding to the first time (*e.g.*, as shown in box b of FIGURE 8) and a second signal signature corresponding to the second time (*e.g.*, as shown in box c of FIGURE 8); and c) analyze the first and second signal signature to determine if transducer motion has occurred (*e.g.*, by estimating a correlation coefficient using a correlation algorithm as shown in box d of FIGURE 8).

Variations of the implementations of the present invention described above will readily occur to those skilled in the art. For instance, the storage device 74, 74' may not be necessary if the sampling of a current motion-indicating signal by circuit 72 or 92 is time-synchronized with the shifting or reading of a reference signal from the storage device 76, 76'. Instead of using separate storage devices 74 and 76 (or 74' and 76'), an embodiment can be implemented using a single storage device. Instead of using a single motion detection element, a subarray of elements can alternatively be used for motion detection. If no motion or only negligible motion has occurred between the beginning of therapy and a later comparison or correlation, the reference signals stored in the reference signal storage device may be updated with current signals. The motion detecting element(s) may be elements of the therapy array, or elements in the probe separate from the therapy array which are dedicated to motion detection. The alert issued by the system when motion is detected may be audible as shown in FIGURE 1, or can be visual or both.

It should be noted that an ultrasound system suitable for use in an implementation of the present invention, and in particular the component structure of the ultrasound system described by FIGURES 1, 6a and 6b, may be implemented in hardware, software or a combination thereof. The various embodiments and/or components of an ultrasound system, for example, the modules, or components and controllers therein, also may be implemented as part of one or more computers or processors. The computer or processor may include a microprocessor. The microprocessor may be connected to a communication bus, for example, to access a PACS system or a data network. The computer or processor may also include a memory. The memory devices such as the storage devices 74, 76, 74' and 76' may include Random Access Memory (RAM) and Read Only Memory (ROM), an analog shift register or an analog random access memory (ARAM), or other digital or analog signal storage components. The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine. In the ultrasound system shown in FIGURES 1, 6a, and 6b for instance, software instructions are conventionally employed by a digital processor to create and control the display and user control functions described above, and to perform analysis such as comparison and correlation coefficient computations. The processor performs analysis in a system of the present invention by executing the correlation algorithms given above, for instance. The present invention is set out in the appended claims. The embodiments, examples or aspects according to the present description which do not fall within the scope of said claims are provided for illustrative purposes only and do not form part of the present invention.

**Claims**

1. An ultrasound system adapted to perform therapy on the head of a subject comprising:

an array (10) of transducer elements, configured to acoustically couple to the head of a subject, and adapted to transmit therapeutic ultrasonic energy toward a therapy site in the head of the subject,
a motion detecting transducer configured to receive echo signals from the head of the subject at a first time and a second time; and **characterized in that** it further comprises
a processor configured to analyze, during therapy, the echo signals received at the first and second times to determine whether transducer motion has occurred from the first time to the second time, wherein the processor further comprises a motion detecting circuit configured to analyze the echo signals received at the first and second times by comparison or correlation.

2. The ultrasound system of Claim 1, wherein the motion detecting transducer further comprises an element of the same array adapted to transmit therapeutic ultrasonic energy.

3. The ultrasound system of Claim 2, wherein the motion detecting transducer further comprises a plurality of transducer elements.

4. The ultrasound system of any of Claims 1 to 3, further comprising a storage device (74, 76; 74', 76') configured to store echo signals received by the motion detecting transducer.

5. The ultrasound system of Claim 4, further comprising a sampling circuit (72) configured to produce analog signal samples of the echo signals received by the motion detecting transducer,
wherein the analog signal samples are stored by the storage device (74, 76).

6. The ultrasound system of Claim 4, wherein the storage device further comprises a digital signal storage device (74', 76').

7. The ultrasound system of Claim 6, further comprising a sampling circuit (92) configured to produce digital signal samples of the echo signals received by the motion detecting transducer,
wherein the digital signal samples are stored by the digital signal storage device (74', 76').

8. The ultrasound system of any of Claims 1 to 7, further comprising a fundamental/harmonic signal separator (22) configured to produce fundamental frequency signals from the echo signals received by the motion detecting transducer.

9. The ultrasound system of any of Claims 1 to 8, wherein the motion detecting circuit is further configured to produce an alert in response to a determination that transducer motion has occurred.

10. The ultrasound system of any of Claims 1 to 9, further comprising a headset (62) configured to maintain the array of transducer elements (10) and the motion detecting transducer in acoustically coupled contact with the head of the subject.

11. The ultrasound system of any of Claims 1 - 10, wherein the array further comprises two backscatter elements (10b, 10c) arranged at opposite sides of the array, wherein the backscatter elements are configured to generate a backscatter signal representative to received backscattered ultrasound from a beam transmission and wherein the processor if further configured to detect the transducer motion based on the backscatter signals.

**Patentansprüche**

1. Ultraschallsystem, das angepasst ist, um eine Therapie am Kopf eines Subjekts durchzuführen, umfassend:

ein Array (10) von Wandlerelementen, das konfiguriert ist, um akustisch an den Kopf eines Subjekts zu koppeln und angepasst ist, um therapeutische Ultraschallenergie zu einer Therapiestelle im Kopf des Subjekts zu übertragen,
einen Bewegungserfassungswandler, der konfiguriert ist, um Echosignale vom Kopf des Subjekts zu einem ersten Zeitpunkt und einem zweiten Zeitpunkt zu empfangen; und **dadurch gekennzeichnet, dass** es ferner einen Prozessor umfasst, der dazu konfiguriert ist, während der Therapie die zum ersten und zweiten Mal empfangenen Echosignale zu analysieren, um zu bestimmen, ob eine Wandlerbewegung vom ersten Mal zum zweiten Mal aufgetreten ist, wobei der Prozessor ferner eine Bewegungserkennungsschaltung umfasst, die konfiguriert ist, um die zum ersten und zweiten Zeitpunkt empfangenen Echosignale durch Vergleich oder Korrelation zu analysieren.

2. Ultraschallsystem nach Anspruch 1, wobei der bewegungsdetektierende Wandler ferner ein Element desselben Arrays umfasst, das angepasst ist, um therapeutische Ultraschallenergie zu übertragen.

3. Ultraschallsystem nach Anspruch 2, wobei der Bewegungserfassungswandler ferner eine Vielzahl von Wandlerelementen umfasst.

4. Ultraschallsystem nach einem der Ansprüche 1 bis 3, das ferner eine Speichervorrichtung (74, 76; 74', 76') umfasst, die konfiguriert ist, um Echosignale zu speichern, die von dem Bewegungserfassungswandler empfangen werden.

5. Ultraschallsystem nach Anspruch 4, das ferner eine Abtastschaltung (72) umfasst, die konfiguriert ist, um analoge Signalabtastwerte der Echosignale zu erzeugen, die von dem Bewegungserfassungswandler empfangen werden, wobei die analogen Signalabtastwerte von der Speichervorrichtung (74, 76) gespeichert werden.

6. Ultraschallsystem nach Anspruch 4, wobei die Speichervorrichtung ferner eine digitale Signalspeichervorrichtung (74', 76') umfasst.

7. Ultraschallsystem nach Anspruch 6, das ferner eine Abtastschaltung (92) umfasst, die konfiguriert ist, um digitale Signalabtastwerte der Echosignale zu erzeugen, die von dem Bewegungserfassungswandler empfangen werden, wobei die digitalen Signalabtastwerte von der digitalen Signalspeichervorrichtung (74', 76') gespeichert werden.

8. Ultraschallsystem nach einem der Ansprüche 1 bis 7, das ferner einen Grundwellen-/Oberwellensignal-Separator (22) umfasst, der konfiguriert ist, um Grundfrequenzsignale aus den von dem Bewegungserfassungswandler empfangenen Echosignalen zu erzeugen.

9. Ultraschallsystem nach einem der Ansprüche 1 bis 8, wobei die Bewegungserfassungsschaltung ferner konfiguriert ist, um als Reaktion auf eine Bestimmung, dass eine Wandlerbewegung aufgetreten ist, eine Warnung zu erzeugen.

10. Ultraschallsystem nach einem der Ansprüche 1 bis 9, das ferner ein Headset (62) umfasst, das konfiguriert ist, um das Array von Wandlerelementen (10) und den Bewegungserfassungswandler in akustisch gekoppeltem Kontakt mit dem Kopf des Subjekts zu halten.

11. Ultraschallsystem nach einem der Ansprüche 1 bis 10, wobei das Array ferner zwei Rückstreuelemente (10b, 10c) umfasst, die an gegenüberliegenden Seiten des Arrays angeordnet sind, wobei die Rückstreuelemente konfiguriert sind, um ein Rückstreusignal zu erzeugen, das für empfangenen rückgestreuten Ultraschall von einer Strahlübertragung repräsentativ ist, und wobei der Prozessor ferner konfiguriert ist, um die Wandlerbewegung basierend auf den Rückstreusignalen zu detektieren.

**Revendications**

1. Système à ultrasons adapté pour effectuer une thérapie sur la tête d'un sujet comprenant:

un réseau (10) d'éléments transducteurs, configuré pour se coupler acoustiquement à la tête d'un sujet, et adapté pour transmettre une énergie ultrasonore thérapeutique vers un site de thérapie dans la tête du sujet, un transducteur de détection de mouvement configuré pour recevoir des signaux d'écho de la tête du sujet à un premier instant et un deuxième instant; et **caractérisé en ce qu'**il comprend en outre un processeur configuré pour analyser, pendant la thérapie, les signaux d'écho reçus aux premier et deuxième instants afin de déterminer si un mouvement du transducteur s'est produit du premier instant au deuxième instant, où le processeur comprend en outre un circuit de détection de mouvement configuré pour analyser les signaux d'écho reçus aux premier et deuxième instants par comparaison ou corrélation.

**2.** Système à ultrasons selon la revendication 1, dans lequel le transducteur de détection de mouvement comprend en outre un élément du même réseau adapté pour transmettre une énergie ultrasonore thérapeutique.

**3.** Système à ultrasons selon la revendication 2, dans lequel le transducteur de détection de mouvement comprend en outre une pluralité d'éléments transducteurs.

**4.** Système à ultrasons selon l'une quelconque des revendications 1 à 3, comprenant en outre un dispositif de stockage (74, 76; 74', 76') configuré pour stocker les signaux d'écho reçus par le transducteur de détection de mouvement.

**5.** Système à ultrasons selon la revendication 4, comprenant en outre un circuit d'échantillonnage (72) configuré pour produire des échantillons de signaux analogiques des signaux d'écho reçus par le transducteur de détection de mouvement,
où les échantillons de signaux analogiques sont stockés par le dispositif de stockage (74, 76).

**6.** Système à ultrasons selon la revendication 4, dans lequel le dispositif de stockage comprend en outre un dispositif de stockage de signaux numériques (74', 76').

**7.** Système à ultrasons selon la revendication 6, comprenant en outre un circuit d'échantillonnage (92) configuré pour produire des échantillons de signaux numériques des signaux d'écho reçus par le transducteur de détection de mouvement,
où les échantillons de signaux numériques sont stockés par le dispositif de stockage de signaux numériques (74', 76').

**8.** Système à ultrasons selon l'une quelconque des revendications 1 à 7, comprenant en outre un séparateur de signaux fondamentaux/harmoniques (22) configuré pour produire des signaux de fréquence fondamentale à partir des signaux d'écho reçus par le transducteur de détection de mouvement.

**9.** Système à ultrasons selon l'une quelconque des revendications 1 à 8, dans lequel le circuit de détection de mouvement est en outre configuré pour produire une alerte en réponse à une détermination qu'un mouvement du transducteur s'est produit.

**10.** Système à ultrasons selon l'une quelconque des revendications 1 à 9, comprenant en outre un casque (62) configuré pour maintenir le réseau d'éléments transducteurs (10) et le transducteur de détection de mouvement en contact couplé acoustiquement avec la tête du sujet.

**11.** Système à ultrasons selon l'une quelconque des revendications 1 à 10, dans lequel le réseau en outre comprend deux éléments de rétrodiffusion (10b, 10c) agencés sur les côtés opposés du réseau, dans lequel les éléments de rétrodiffusion sont configurés pour générer un signal de rétrodiffusion représentatif des ultrasons rétrodiffusés reçus à partir d'une transmission de faisceau et dans lequel le processeur est en outre configuré pour détecter le mouvement du transducteur sur la base des signaux de rétrodiffusion.

FIG. 1

EP 3 554 378 B1

FIG. 2

FIG. 3

62 —
14
12
60

# FIG. 4

70

# FIG. 5

FIG. 6a

FIG. 6b

```
┌─────────────────────────────────────┐
│  Position headset on head of patient │──44
└─────────────────────────────────────┘
                   │
                   ▼
┌─────────────────────────────────────┐
│         Start therapy procedure      │──46
└─────────────────────────────────────┘
                   │
                   ▼
┌─────────────────────────────────────┐
│      Store signals from transducer in│──48
│         reference storage device     │
└─────────────────────────────────────┘
                   │
                   ▼
┌─────────────────────────────────────┐
│     Periodically acquire signals from│
│      transducer, compare/correlate   │──52
│         with reference signals       │
└─────────────────────────────────────┘
                   │
                   ▼
┌─────────────────────────────────────┐
│    Issue alert when instant & referen│ce
│      signals do not compare/correlate│──54
└─────────────────────────────────────┘
```

# FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8211023 B, Swan
- WO 2005074805 A, Bruce
- US 6530885 B, Entrekin
- US 6723050 B, Dow
- US 5181514 A, Solomon
- US 5720291 A, Schwartz